# EUROPÄISCHE PATENTSCHRIFT

(11) **EP 0 549 621 B1**
(45) Veröffentlichungstag und Bekanntmachung des Hinweises auf die Patenterteilung: **27.09.1995**
(21) Anmeldenummer: 91915659.6
(22) Anmeldetag: 28.08.1991
(51) Int. Cl.: C07C 59/64, C07C 59/68, C07C 59/90, C07C 65/40, C07D 213/55, A61K 31/19, A61K 31/44

(54) **NEUE LEUKOTRIEN-B4-ANTAGONISTEN, VERFAHREN ZU IHRER HERSTELLUNG UND IHRE VERWENDUNG ALS ARZNEIMITTEL**
NEW LEUKOTRIENE-B4 ANTAGONISTS, PROCESS FOR PRODUCING THE SAME AND THEIR USE AS MEDICINAL DRUGS
NOUVEAUX ANTAGONISTES DES LEUCOTRIENES B4, LEUR PROCEDE DE PRODUCTION ET LEUR UTILISATION COMME MEDICAMENTS

(30) Priorität: 07.09.1990 DE 4028866
(43) Veröffentlichungstag der Anmeldung: 07.07.1993
(73) Patentinhaber: SCHERING AKTIENGESELLSCHAFT, 13342 Berlin (DE)
(72) Erfinder: HEINDL, Josef, D-1000 Berlin 38 (DE); SKUBALLA, Werner, D-1000 Berlin 28 (DE); BUCHMANN, Bernd, D-1000 Berlin 21 (DE); FRÖHLICH, Wolfgang, D-1000 Berlin 31 (DE); EKERDT, Roland, D-1000 Berlin 28 (DE); GIESEN, Claudia, D-1000 Berlin 20 (DE)
(86) Internationale Anmeldenummer: DE9100690
(87) Internationale Veröffentlichungsnummer: WO9204311

(56) Entgegenhaltungen:
- EP-A- 0 108 592
- EP-A- 0 132 366
- EP-A- 0 132 367
- EP-A- 0 150 166
- EP-A- 0 276 064
- US-A- 4 567 184

## Beschreibung

Die Erfindung betrifft neue Leukotrien-B₄-Antagonisten, Verfahren zu ihrer Herstellung sowie ihre Verwendung als Arzneimittel.
Leukotrien B₄ (LTB₄) wurde 1979 von B. Samuelsson und Mitarbeiter als Metabolit der Arachidonsäure entdeckt. Bei der Biosynthese wird durch das Enzym 5-Lipoxygenase zunächst als zentrales Zwischenprodukt das Leukotrien A₄ gebildet, das dann durch eine spezifische Hydrolase in das LTB₄ umgewandelt wird. 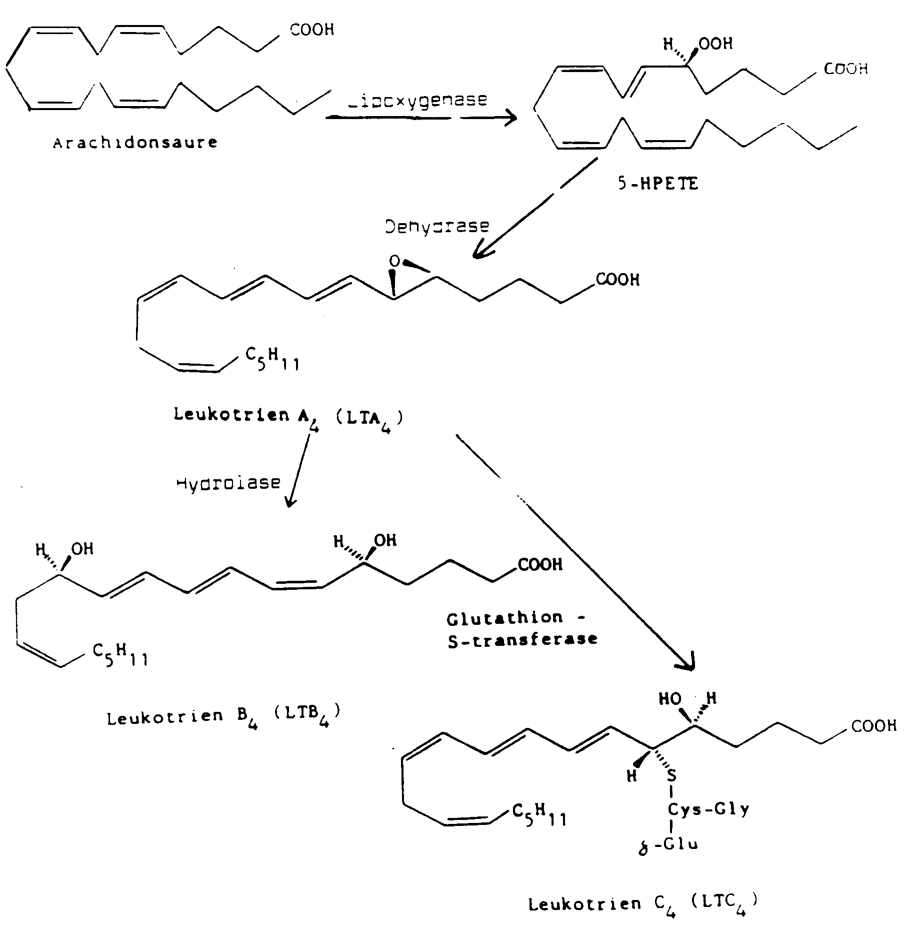
Die Nomenklatur der Leukotriene kann folgenden Arbeiten entnommen werden: a) B. Samuelsson et al., Prostaglandins 19, 645 (1980); 17, 785 (1979). b) C.N. Serhan et al., Prostaglandins 34, 201 (1987).

Die physiologische und insbesondere die pathophysiologische Bedeutung des Leukotrien B₄ wird in einigen neueren Arbeiten zusammengefaßt: a) The Leukotrienes, Chemistry and Biology eds. L.W. Chakrin, D.M. Bailey, Academic Press 1984. b) J.W. Gillard et al., Drugs of the Future 12, 453 (1987). c) B. Samuelsson et al., Science 237, 1171 (1987). d) C.W. Parker, Drug Development Research 10, 277 (1987). Hieraus ergibt sich, daß LTB₄ ein wichtiger Entzündungsmediator für entzündliche Erkrankungen ist, bei denen Leukozyten in das erkrankte Gewebe einwandern.
Von LTB₄ weiß man, daß es die Adhäsion von Leukozyten an die Blutgefäßwand verursacht. LTB₄ ist chemotaktisch wirksam, d.h.es löst eine gerichtete Wanderung von Leukozyten in Richtung eines Gradienten steigender Konzentration aus. Außerdem verändert es aufgrund seiner chemotaktischen Aktivität indirekt die vasculäre Permeabilität, wobei ein Synergismus mit Prostaglandin E₂ beobachtet wird. LTB₄ spielt offensichtlich bei entzündlichen, allergischen und immunologischen Prozessen eine entscheidende Rolle.

Leukotriene und insbesondere LTB₄ sind an Hauterkrankungen beteiligt, die mit entzündlichen Prozessen (erhöhte Gefäßpermeabilität und Ödembildung, Zellinfiltration), erhöhter Proliferation der Hautzellen und Juckreiz einhergehen, wie beispielsweise bei Ekzemen, Erythemen, Psoriasis, Pruritus und Akne. Pathologisch erhöhte Leukotrien-Konzentrationen sind an der Entstehung vieler Dermatitiden entweder ursächlich beteiligt, oder es besteht ein Zusammenhang zwischen der Persistenz der Dermatitiden und den Leukotrienen. Deutlich erhöhte Leukotrienkonzentrationen wurden beispielsweise in der Haut von Patienten mit Psoriasis oder atopischer Dermatitis gemessen.
Weiterhin sind Leukotriene und LTB₄ insbesondere bei Arthritis, chronischer Lungenerkrankungen (z.B. Asthma), Rhinitis und entzündlichen Darmerkrankungen beteiligt.

Antagonisten gegen LTB₄ selbst oder Inhibitoren jener Enzyme, die an der Synthese des LTB₄ beteiligt sind, können als spezifische Heilmittel, besonders gegen Krankheiten, die mit Entzündungen und allergischen Reaktionen einhergehen, wirksam sein.

Aus EP276064 sind bereits Verbindungen mit einer Carboxybenzolphenylpropionsäure-Struktur, die Leukotrien-D₄- und Leukotrien-B₄- antagonistische Eigenschaften besitzen, bekannt.

Es wurden Verbindungen gefunden, die die Wirkung des natürlichen LTB₄ überraschend stark antagonisieren.

Die Erfindung betrifft Leukotrien-B₄-Antagonisten der Formel I, 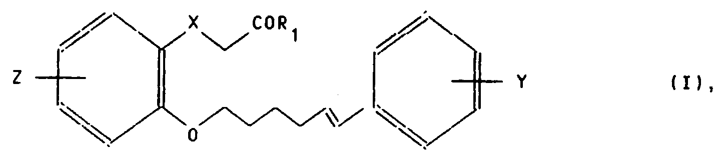
worin
- X: eine CH₂-Gruppe oder ein Sauerstoffatom,
- Y: C₁-C₄-Alkoxy oder -S(O)ₚ-(C₁-C₄)-Alkyl,
- p: 0, 1 oder 2
- Z: ein Wasserstoffatom oder den Rest-A-B-COOH mit A in der Bedeutung einer Hydroxymethylengruppe oder einer Carbonylgruppe und B in der Bedeutung einer Alkylengruppe mit 1 - 6 C-Atomen in der Kette oder eines Restes 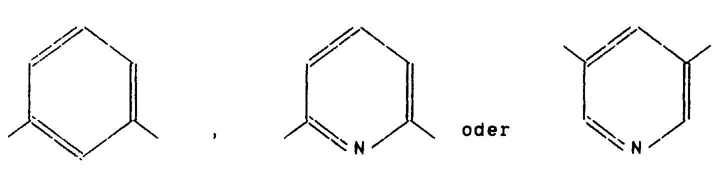 mit der Ausnahme, daß B nicht den Rest 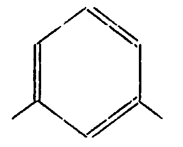
bedeutet, wenn X eine CH₂-Gruppe darstellt, R₁ den Rest OH, -O-(C₁-C₄)-Alkyl, -O-(C₃-C₆)-Cycloalkyl,-O-(C₆-C₁₀)-Aryl, -O-(C₇-C₁₂)-Aralkyl oder den Rest NR₄R₆ mit R₄ in der Bedeutung Wasserstoff, (C₁-C₄)-Alkyl, (C₃-C₆)-Cycloalkyl oder (C₇-C₁₂)-Aralkyl und R₆ in der Bedeutung (C₁-C₄)-Alkyl, (C₃-C₆)-Cycloalkyl oder (C₇-C₁₂)-Aralkyl darstellen sowie deren Salze mit physiologisch Verträglichen Basen und deren Cyclodextrinclathrate.

Y und R₁ als C₁-C₄-Alkoxy-Gruppe können bedeuten: Methoxy, Ethoxy, n-Propoxy, Isoproxy, n-Butoxy, sek.-Butoxy und tert.-Butoxy.

Der C₁-C₄-Alkylrest in der Gruppe -S(O)ₚ-(C₁-C₄)-Alkyl von Y bzw. als Rest R oder R₄ kann sein: Methyl, Ethyl, n-Propyl-Isopropyl, n-Butyl, sek.-Butyl, tert.-Butyl.

Als Alkylengruppe Z mit 1-6 C-Atomen kommen geradkettige oder verzweigtkettige, gesättigte Reste in Betracht, wie z. B. methylen, ethylen, trimethylen, tetramethylen, hexamethylen, 1-methyltrimethylen, 1-methyl-tetramethylen, 1,1-dimethyl-trimethylen usw.

Der Rest (C₃-C₆)-Cycloalkyl (für R₁ und R₄) kann sein: Cyclopropyl, Cyclobutyl, Cyclopentyl oder Cyclohexyl.

Als Reste C₆-C₁₀-Aryl in der Definition von R₁ kommen Phenyl, 1-Naphthyl, 2-Naphthyl in Betracht.

Die Reste C₇-C₁₂-Aralkyl in den Definitionen von R₁ und R₄ schließlich stellen die folgenden Gruppen dar: Benzyl, Phenethyl, 3-Phenylpropyl, 4-Phenylbutyl, 1-Methyl-3-phenylpropyl, 1-Methyl-2-phenyl-ethyl usw.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natrium- und Kaliumhydroxid, Erdalkalihydoxide, wie Calciumhydroxid, Amoniak, Amine, wie Ethanolamin, Diethanolamin, Triethanolamin, N-Methylglucamin, Morphin, Tris-(hydroxymethyl)-methylamin usw.

Um zu den Cyclidextrinclathraten zu gelangen, werden die Verbindungen der Formel I mit α-, β- oder γ-Cyclodextrin umgesetzt, Bevorzugt sind die β-Cyclodextrinclathrate.

Die Erfindung betrifft außerdem ein Verfahren zur Herstellung von Leukotrien-B₄-Antagonisten der Formel I, dadurch gekennzeichnet, daß man in an sich bekannter Weise eine Verbindung der Formel II, 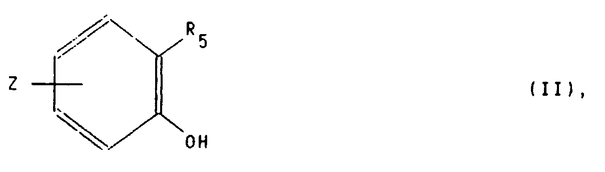
worin Z die oben angegenene Bedeutung hat und R₅ die Reste OH oder X-CH₂-COOR bedeutet, wobei X die oben angegebene Bedeutung hat und R eine C₁-C₄-Alkylgruppe darstellt, mit einer Verbindung der Formel III, 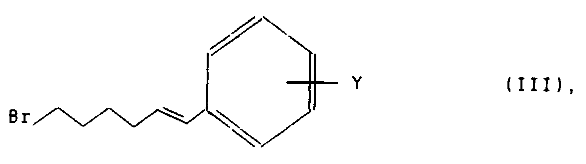
worin Y die oben angegebene Bedeutung hat, in Gegenwart von Cäsium-, Lithium- oder Kaliumcarbonat umsetzt und für den Fall, daß R₅ den Rest OH bedeutet mit einer Verbindung der Formel IV,

BrCH₂COOR (IV),

worin R die oben angegebene Bedeutung hat, umsetzt, gegebenenfalls die Carbonylgruppe A reduziert, Estergruppen verseift, Carboxylgruppen verestert oder die erhaltenen Sauren der Formel I mit organischen oder anorganischen Basen oder Cyclodextrinen umsetzt.

Das oben genannte Verfahren (II+III-I) wird in organischen Losungsmitteln, wie z. B. Dimethylformamid, bei Temperaturen zwischen 0 und 100 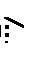 C, vorzugsweise bei Temperaturen zwischen 20 und 60 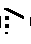 C unter Rühren im Verlaufe von 5-24 Stunden in Gegenwart von Cäsium-, Lithium-oder Kaliumcarbonat durchgeführt.

Die Reduktion der Carbonylgruppe A erfolgt vorzugsweise mit Natriumborhydrid unter den üblichen Bedingungen. Die erhaltenen Hydroxymethylenverbindungen können gewünschtenfalls in die optischen Antipoden aufgetrennt werden.

Die Herstellung der für diese Umsetzung benötigten Verbindungen der Formel II und III erfolgt nach den in den Beispielen, bzw. in den Referenzbeispielen angegebenen Verfahren.

Die Verseifung der Ester der Formel I wird nach den dem Fachmann bekannten Methoden durchgeführt, wie beispielsweise mit basischen Katalysatoren.

Die Einführung der Estergruppe 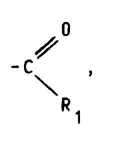
bei welcher R₁ eine O-Alkylgruppe mit 1-4 C-Atomen darstellt, erfolgt nach den dem Fachmann bekannten Methoden. Die l-Carboxy-verbindungen werden beispielsweise mit Diazokohlenwasserstoffen in an sich bekannter Weise umgesetzt. Die Veresterung mit Diazokohlenwasserstoffen erfolgt z. B. dadurch, daß man eine Lösung des Diazokohlenwasserstoffes in einem inerten Lösungsmittel, vorzugsweise in Diethylether, mit der 1-Carboxyverbindung in dem gleichen oder in einem anderen inerten Lösungsmittel, wie z. B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org. Reactions Bd. 8, Seiten 389 - 394 (1954)].

Die Einführung der Estergruppe 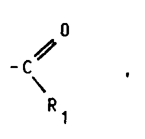
bei welcher R¹ eine -0-Arylgruppe darstellt, erfolgt nach den dem Fachmann bekannten Methoden. Beispielsweise werden die 1-Carboxyverbindungen mit den entsprechenden Arylhydroxyverbindungen mit Dicyclohexylcarbodiimid in Gegenwart einer geeigneten Base, beispielsweise Pyridin, DMAP, Triethylamin, in einem inerten Lösungsmittel umgesetzt, Als Lösungsmittel kommen Methylenchlorid, Ethylenchlorid, Chloroform, Essigester, Tetrahydrofuran, vorzugsweise Chloroform in Frage. Die Reaktion wird bei Temperaturen zwischen -30 °C und +50 °C, Vorzugsweise bei 10 °C, durchgeführt.

Die Leukotrien-B₄-Antagonisten der Formel I mit R¹ in der Bedeutung einer COOH-Gruppe können mit geeigneten Mengen der entsprechenden anorganischen Basen unter Neutralisierung in ein Salz überführt werden. Beispielsweise erhält man Dein Lösen der entsprechenden Säuren in Wasser, das die stöchiometrische Menge der Base erhält, nach Abdampfen des Wassers oder nach Zugabe eines mit Wasser mischbaren Lösungsmittels, z. B. Alkohol oder Aceton, das feste anorganische Salz.

Zur Herstellung eines Aminsalzes wird die LTB₄-Säure z. B. in einem geeigneten Lösungsmittel, beispielsweise Ethanol, Aceton, Diethylether, Acetonitril oder Benzol gelöst und mindestens die stöchionetrische Menge des Amins dieser Lösung zugesetzt, Dabei fällt das Salz gewöhnlich in fester Form an oder wird nach Verdampfen des Lösungsmittels in üblicher Weise isoliert.

Die Einführung der Amidgruppe 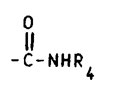
erfolgt nach den dem Fachmann bekannten Methoden. Die Carbonsäuren der Formel I (R₁=OH) werden zunächst in Gegenwart eines tertiären Amins, wie beispielsweise Triethylamin, mit Chlorameisensäureisobutylester in das gemischte Anhydrid überführt. Die Umsetzung des gemischten Anhydrids mit dem Alkalisalz des entsprechenden Amins oder mit Ammoniak erfolgt in einem inerten Lösungsmittel oder Lösungsmittelgemisch, wie beispielsweise Tetrahydrofuran, Dimethoxyethan, Dimethylformamid, Hexamethylphosphorsäuretriamid, bei Temperaturen zwischen -30°C und +60°C, vorzugsweise bei 0°C bis 30°C.

Die Verbindungen der Formel I wirken antientzündlich und antiallergisch. Folglich stellen die neuen Leukotrien B₄-Derivate der Formel I wertvolle pharmazeutische Wirkstoffe dar. Die Verbindungen der Formel I sind besonders zur topischen Applikation geeignet, da sie eine Dissoziation zwischen erwünschter topischer Wirksamkeit und unerwünschten systemischen Nebenwirkungen aufweisen.

Die neuen Leukotrien B₄-Antagonisten der Formel I eignen sich in Kombination mit den in der galenischen Pharmazie üblichen Hilfs- und Trägermitteln zur lokalen Behandlung von Kontaktdermatitis, Ekzemen der verschiedensten Art, Neurodermatosen, Erytrodermie, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigneten Zusätzen in die gewünschte Applikationsform, wie z. B.: Lösungen, Lotionen, Salben, Cremes oder Pflaster, überführt.
In den so formulierten Arzneimitteln ist die Wirkstoffkonzentration von der Applikationsform abhängig. Bei Lotionen und Salben wird vorzugsweise eine Wirkstoffkonzentration von 0,0001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Leukotrien-B₄-Antagonisten auch in Form von Kapseln, Tabletten oder Dragees, die vorzugsweise 0,1 bis 100 mg Wirkstoff enthalten oder oral appliziert werden oder in Form von Suspensionen, die vorzugsweise 1-200 mg Wirkstoff pro Dosiseinheit enthalten und rektal appliziert werden auch zur Behandlung allergischer Erkrankungen des Darmtraktes, wie der Kolitis ulcerosa und der colitis granulomatosa.

Die neuen Leukotrien-B₄-Derivate können auch in Kombination, wie z.B mit Lipoxygenasehemmern, Cyclooxygenasehemmern, Prostacyclinagonisten, Prostaglandinagonisten, Thromboxanantagonisten, Leukotrien D₄-antagonisten, Leukotrien-E₄-antagonisten, Leukotrien F₄-antagonisten, Phosphodiesterasehemmern, Calciumantagonisten oder PAF-Antagonisten verwendet werden.

### Referenzbeispiele

1) (5E,Z)-6-(4-Methoxyphenyl)-5-hexensäuremethylester
   Zu 97,7 g Carboxybutyltriphenylphosphoniumbromid in 205 ml Dimethylsulfoxid/108 ml Tetrahydrofuran gibt man portionsweise unter Argon bei 0°C insgesamt 49,6 g Kalium-tert.-butylat und rührt 1 Stunde bei 0°C. Dann tropft man 11,2 g 4-Methoxybenzaldehyd gelöst in 200 ml Tetrahydrofuran zu und rührt 2,5 Stunden bei 50°C. Anschließend tropft man 62,7 g Methyljodid gelöst in 50 ml Tetrahydrofuran zu und rührt 16 Stunden bei 24°C. Man gießt die Reaktionsmischung auf 800 ml Eiswasser, extrahiert dreimal mit je 400 ml Methylenchlorid, trocknet über Natriumsulfat und dampft im Vakuum ein. Den Rückstand reinigt man durch eine Flash-Chromatographie an Kieselgel mit Hexan/10-20% Essigester. Man erhält 15,8 g der Titelverbindung als farbloses Öl.
   IR: 3005, 2958, 2842, 1730, 1608, 1510, 1440, 1248, 1175, 1033, 968, 842cm⁻¹
2) (5E)-6-(4-Methoxyphenyl)-5-hexen-1-ol
   Zu 15,7 g des vorstehend hergestellten Esters in 490 ml Toluol tropft man unter Argon bei -70°C 139 ml (168mmol) einer DIBAH-Lösung in Toluol und läßt innerhalb von 2 Stunden auf -10°C erwärmen. Dann gibt man vorsichtig bei -70°C 56 ml Isopropanol gefolgt von 80 ml Wasser zu und rührt 2 Stunden bei 24°C. Man filtriert vom Niederschlag ab und wäscht gut mit Essigester nach. Den so erhaltenen Rückstand löst man in 800 ml Dimethylsulfoxid/Tetrahydrofuran (1+1), versetzt unter Argon bei 24°C mit 24 g Kalium-tert.-butylat und rührt 15 Stunden bei 24°C. Anschließend gibt man auf 1000 ml Eiswasser, extrahiert fünfmal mit Ether, wäscht die vereinigten organischen Phasen mit Sole, trocknet über Magnesiumsulfat und dampft im Vakuum ein. Den Rückstand reinigt man durch eine Flash-Chromatographie an Kieselgel mit Hexan/ 20-50% Essigester. Man erhält ein vorgereinigtes Produkt, welches durch Umkristallisation aus Hexan/Toluol nochmals gereinigt wird. Man erhält 9,6 g der Titelverbindung als farblose Kristalle. Schmpkt. 66°C.
   IR: 3625, 3460, 3008, 2940, 2842, 1610, 1512, 1245, 1176, 1037, 968 cm⁻¹.
3) (1E)-6-Brom-1-(4-methoxyphenyl)-1-hexen
   Zu einer Losung von 4,18 g des vorstehend hergestellten Alkohols in 200 ml Methylenchlorid gibt man bei -78°C unter Argon 2ml Pyridin, 6,62 g Tetrabrommethan und 5,0 g Triphenylphosphin. Unter Rühren läßt man innerhalb von 1,5 Stunden auf 24°C erwärmen. Man dampft im Vakuum ein und chromatographiert den Rückstand an Kieselgel mit Hexan/ 10-20% Essigester. Man erhält 5,2 g der Titelverbindung als farbloses Öl.
   IR: 3000, 2970, 2938, 1610, 1512, 1438, 1247, 1105, 968 cm⁻¹.

### Beispiel 1

### 3-[2-[6-(4-Methoxyphenyl)-(5E)-5-hexenyloxy]-phenyl]-propionsäure

A. Zu einer Losung von 180 mg 3-(2-Hydroxyphenyl)-propionsäuremethylester und 272 mg (1E)-6-Brom-1-(4-methoxyphenyl)-1-hexen in 2 ml Dimethylformamid werden 651 mg Cäsiumcarbonat gegeben und die Suspension 16 Stunden bei Raumtemperatur gerührt. Die Reaktionsmischung wird filtriert, der Filterrückstand mit Dichlormethan gewaschen, das Filtrat eingeengt und der Rückstand an Kieselgel mit Hexan chromatographiert. Es werden 255 mg 3-[2-[6-(4-Methoxyphenyl)-(5E)-5-hexenyloxy]-phenyl]propionsäuremethylester als Ol erhalten.
   IR (CHCl₃): 2942, 1730, 1610, 1512, 1498, 1241, 1110, 1030, 969 cm⁻¹
B. Eine Lösung von 100 mg 3-[2-[6-(4-Methoxyphenyl)-(5E)-5-hexenyloxyphenyl]-propionsäuremethylester in 2 ml Methanol und 2 ml 1n Kalilauge wird 2 1/2 Stunden bei Raumtemperatur gerührt. Die Reaktionsmischung wird mit 2 n Salzsäure auf pH 1 angesäuert und mit Ethylacetat ausgeschüttelt, die organische Phase über Natriumsulfat getrocknet und eingeengt. Es werden 92 mg der Titelverbindung als Ol erhalten.
   IR (CHCl₃): 2940, 1710, 1608, 1510, 1498, 1245, 1100, 1015, 968 cm⁻¹.

### Beispiel 2

### 5-(3-Carboxybenzoyl)-2-[6-(4-methoxyphenyl)-(5E)-5-hexenyloxy]-phenoxyessigsäure

A. Zu einer Lösung von 7,34 g Isophthalsäuremonomethylesterchlorid in 160 ml Dichlormethan werden unter Eiskühlung 14,8 g Aluminiumchlorid und 5 g Veratrol gegeben. Die Reaktionsmischung wird 8 Stunden unter Rückfluß erhitzt, auf Eiswasser gegossen, mit 2 n Salzsäure angesäuert und mit Dichlormethan ausgeschüttelt. Die organische Phase wird über Natriumsulfat getrocknet, eingeengt und der Rückstand an Kieselgel mit Hexan/Ethylacetat = 8/2 chromatographiert. Es werden 3,5 g 3-(4-Hydroxy-3-methoxybenzoyl)-benzoesäuremethylester vom Schmelzpunkt 133-134°C erhalten.
   IR (CHCl₃): 3520, 2945, 1715, 1644, 1590, 1500, 1423, 1310, 1265 cm⁻¹.
B. 3,4 g 3-(4-Hydroxy-3-methoxybenzoyl)-benzoesäuremethylester werden mit 43 g Pyridin-Hydrochlorid vermischt und 4 Stunden unter Rühren auf 180°C erhitzt. Die Reaktionsmischung wird nach Abkühlen auf 90°C mit Wasser versetzt, mit Dichlormethan ausgeschüttelt, über Natriumsulfat getrocknet und eingeengt. Es werden 2,9 g 3-(3,4-Dihydroxy-benzoyl)-3-(3,4-Dihydroxy-benzoyl)-benzoesäure vom Schmelzpunkt 254-256°C erhalten.
   IR (KBr): 3510, 3300, 1740, 1700, 1640, 1590, 1579, 1440, 1318, 1240, 1120, 740, 715, 615 cm⁻¹.
C. Eine Lösung von 2,9 g 3-(3,4-Dihydroxybenzoyl)-benzoesäure in 100 ml Methanol wird mit 7 g Amberlyst 15® versetzt und 8 Stunden unter Rühren zum Rückfluß erhitzt. Die Reaktionsmischung wird über Kieselgur filtriert, das Filtrat eingeengt, in Ethylacetat aufgenommen und mit 10%iger Natriumhydrogencarbonatlösung ausgeschüttelt, Die organische Phase wird über Natriumsulfat getrocknet und eingeengt. Es werden 1,4 g 3-(3.4-Dihydroxybenzoyl)-benzoesäuremethylester als öliges Rohprodukt erhalten. 272 mg dieses Rohprodukts werden in 2,5 ml Dimehtylformamid gelöst, mit 269 mg (1E)-6-Brom-1-(4-methoxyphenyl)-1-hexen und 185 mg Lithiumcarbonat versetzt und die Mischung unter Rühren 18 Stunden auf 50-60°C erhitzt. Die Reaktionsmischung wird filtriert, mit Dichlormethan gewaschen, das Filtrat eingeengt und der Rückstand an Kieselgel mit Hexan/Ethylacetat = 98/2 chromatographiert. Es werden 230 mg 3-[3-Hydroxy-4-[6-(4-methoxyphenyl)-(5E)-5-hexenyloxy]-benzoyl]-benzoesäuremethylester als Öl erhalten.
   IR (CHCl₃): 3670, 3535-3330, 2995, 2935, 1723, 1670, 1610, 1500, 1386, 1255, 1091, 970 cm⁻¹.
D. Unter den Bedingungen des Beispiels 1A werden 240 mg 3-[3-Hydroxy-4-[6-4-methylphenyl)-(5E)-5-hexenyloxy]-benzoyl]-benzoesäuremethylester mit 87 mg Bromessigsäureethylester umgesetzt, aufbereitet und an Kieselgel mit Hexan/Ethylacetat = 8/2 chromatographiert. Es werden 120 mg 5-(3-Methoxycarbonyl-benzoyl)-2-[6-(4-methoxyphenyl)-(5E)-5-hexenyloxy] -phenoxyessigsäureethylester als farbloses Öl erhalten. IR (Film): 2950, 2840, 1758, 1725, 1653, 1598, 1512, 1430, 1309, 1250, 1138, 1034, 968, 741, 724 cm⁻¹.
E. Unter den Bedingungen des Beispiels 1B werden 30 mg 5-(3-Methoxycarbonylbenzoyl)-2-[6-(4-methoxyphenyl)-(5E)-5-hexenyloxy]-phenoxyessigsäureethylester in 1 ml Methanol mit 1 ml 1 n Natronlauge verseift und aufbereitet. Es werden 22 mg 5-(3-Carboxybenzoyl)-2-[6-(4-methoxyphenyl]-(5E)-5-hexenyloxy)-phenoxyessigsäure als farbloses Ol erhalten.
   IR (CHCl₃): 2960, 1728, 1603, 1510, 1260, 1095, 1011 cm⁻¹.

### Beispiel 3

### 5-[3-(2-Carboxyethyl)-4-[6-(4-methoxyphenyl)-(5E)-5-hexenyloxy]-phenyl]-5-oxopentansäure

A. Zu einer Suspension von 10 g Aluminiumchlorid in 100 ml Dichlormethan werden unter Eiskühlung nacheinander 5,1 g Glutarsäuremonomethylesterchlorid und 5g 3-(2-Methoxyphenyl)-propionsäuremethylester gegeben und die Mischung 16 Stunden bei Raumtemperatur gerührt. Die Reaktionsmischung wird auf Eis/ 2 n Salzsäure gegossen mit Dichlormethan ausgeschüttelt, die organische Phase über Natriumsulfat getrocknet und eingeengt. Der Rückstand wird am Kieselgel mit Hexan/Ethylacetat (0-30% Ethylacetat) chromatographiert. Es werden 4,45 g 5-⁸4-Methoxy-3-(2-methoxycarbonylethyl)-phenyl]-5-oxo-pentansäuremethylester als Öl erhalten, das zur weiteren Umsetzung zusammen mit 47,5 g Pyridinhydrochlorid 5 Stunden auf 180°C erhitzt wird. Nach Abkühlen auf 90°C wird die Reaktionsmischung mit Wasser versetzt, mit konzentrierter Salzsäure auf pH1 angesäuert, mit Ethylacetat ausgeschüttelt, die organische Phase über Natriumsulfat getrocknet und eingeengt. Es werden 3,9 g 5-[3-(2-Carboxyethyl)-4-hydroxyphenyl]-5-oxopentansäure vom Schmelzpunkt 169-166°C erhalten.
   IR (KBr): 3370, 1700, 1660, 1590, 1282, 1154, 1118 cm⁻¹.
B. Eine Lösung von 3,7 g 5-[3-(2-Carboxyethyl)-4-hydroxyphenyl]-5-oxopentansäure in 190 ml Methanol wird mit 7,7 g Amberlyst 15® versetzt und 24 Stunden bei Raumtemperatur gerührt. Die Reaktionsmischung wird über Kieselgur filtriert, das Filtrat eingeengt, in Ethylacetat aufgenommen und mit 10%iger Natriumhydrogencarbonatlösung ausgeschüttelt. Die organische Phase wird über Natriumsulfat getrocknet, eingeengt und der Rückstand über Kieselgel mit Hexan/Ethylacetat (5-35% Ethylacetat) chromatographiert. Es werden 3,38g 5-[4-Hydroxy-3-(2-methoxycarbonylethyl)-phenyl]-5-oxo-pentansäuremethylester als Öl erhalten.
   IR (Film): 3700-3040, 2950, 1730, 1715, 1670, 1595, 1505, 1435, 1360, 1280, 1163, 1118, 1015 cm⁻¹.
C. Unter den Bedingungen des Beispiels 1A. werden 500 mg 5-[4-Hydroxy-3-(2methoxycarbonylethyl)-phenyl]-5-oxo-pentansäuremethylester mit 436 mg (1E)-6-Brom-1-(4-methoxyphenyl)-1-hexen umgesetzt, aufbereitet und das Rohprodukt an Kieselgel mit Hexan/Ethylacetat (0-15 % Ethylacetat) chromatographiert. Es werden 657 mg 5-[3-(2-Methoxycarbonylethyl)-4-[6-(4-methoxyphenyl)-(5E)-5-hexenyloxy]-phenyl]-5-oxo-pentansäuremethylester als Öl erhalten.
   IR (CHCl₃): 2955, 1732, 1675, 1602, 1510, 1440, 1248, 1185, 1120, 1034, 968 cm⁻¹.
D. Eine Lösung von 400 mg 5-[3-(2-Methoxycarbonylethyl)-4-[6-(4-methoxyphenyl)(5E)-5-hexenyloxy]-phenyl]-5-oxo-pentansäuremethylester in 33 ml Methanol und 13 ml 1 n Natronlauge wird 16 Stunden bei Raumtemperatur gerührt. Das Methanol wird im Vakuum abdestilliert, die alkalische Lösung mit 1 n Salzsäure auf pH 1 angesäuert mit Ethylacetat ausgeschüttelt, die organische Phase über Natriumsulfat getrocknet und eingeengt. Es werden 272 mg der Titelverbindung vom Schmelzpunkt 113-114° erhalten.
   IR (CHCl₃): 3685, 3600-3140, 2940, 1713, 1680, 1607, 1512, 1250, 1180, 970 cm⁻¹.

### Beispiel 4

### 3-{5-[6-Carboxy-2-pyridylcarbonyl]-2-[6-(4-methoxyphenyl)-(5E)-5-hexenyloxy]-phenyl}-propionsäure

A. Zu einer Suspension von 4,4 g Aluminiumchlorid in 50 ml Dichlormethan werden unter Eiskühlung nacheinander 2,2 g 6-Methoxycarbonyl-pyridin-2-carbonsäurechlorid und 1,0 g 3-(2-Methoxyphenyl)-propionsäuremethylester gegeben und die Mischung 16 Stunden bei Raumtemperatur gerührt. Die Reaktionsmischung wird auf Eis/2n Salzsäure gegossen, mit Dichlormethan ausgeschüttelt, die organische Phase über Natriumsulfat getrocknet und eingeengt. Der Rückstand wird an Kieselgel mit Hexan/Ethylacetat (0-15 %) chromatographiert.
   Es werden 1,32 g 3-[5-(6-Methoxycarbonyl-2-pyridylcarbonyl)-2-methoxyphenyl]-propionsäuremethylester vom Schmelzpunkt 97-98 °C erhalten.
   IR (CHCl₃): 2960, 1730, 1655, 1600, 1330, 1260, 1120, 1012 cm⁻¹.
B. 1,3 g 3-[5-(6-Methoxycarbonyl-2-pyridylcarbonyl)-2-methoxy-phenyl]-propionsäuremethylester werden in 12 ml 62 %iger Bromwasserstoffsäure 12 Stunden unter Rückfluß erhitzt. Die Reaktionsmischung wird auf Eis/Wasser gegossen, der Niederschlag abgesaugt und getrocknet. Das Rohprodukt (1,2 g) wird in 11 ml Methanol mit 3 Tropfen konzentrierter Schwefelsäure 6 Stunden unter Rückfluß erhitzt und eingeengt. Der Rückstand wird in Ethylacetat aufgenommen, mit 10 %iger Natriumhydrogencarbonatlösung ausgeschüttelt, mit gesättigter Kochsalzlösung gewaschen, über Natriumsulfat getrocknet und eingeengt. Der Rückstand wird an Kieselgel mit Hexan/Ethylacetat (0-40 %) chromatographiert. Es werden 640 mg 3-[5-(6-Methoxycarbonyl-2-pyridylcarbonyl)-2-hydroxyphenyl]-propionsäuremethylester vom Schmelzpunkt 121-122 °C erhalten.
   IR (CHCl₃): 3460-3120, 2945, 1720, 1650, 1598, 1580, 1435, 1225, 1133 cm⁻¹.
C. Unter den Bedingungen des Beispiels 1 A werden 640 mg 3-[5-(6-Methoxycarbonyl-2-pyridylcarbonyl)-2-hydroxy-phenyl]-propionsäuremethylester mit 505 mg (1E)-6-Brom-1-(4-methoxyphenyl)-1-hexen umgesetzt, aufbereitet und das Rohprodukt an Kieselgel mit Hexan/Ethylacetat (0-15 %) chromatographiert. Es werden 732 mg 3-{5-[6-Methoxycarbonyl-2-pyridylcarbonyl]-2-[6-(4-methoxyphenyl)-(5E)-5-hexenyloxy]-phenyl}-propionsäuremethylester vom Schmelzpunkt 80-82 °C erhalten.
   IR (CHCl₃): 2940, 1722, 1650, 1595, 1500, 1433, 1325, 1240, 1110 cm⁻¹.
D. Unter den Bedingungen des Beispiels 3 D werden 65 mg 3-{5-[6-Methoxycarbonyl-2-pyridylcarbonyl]-2-[6-(4-methoxyphenyl)-(5E)-5-hexenyloxy]-phenyl}-propionsäuremethylester in 2 ml Methanol mit 1 ml Natronlauge verseift und aufbereitet. Es werden 42 mg der Titelverbindung vom Schmelzpunkt 163-166 °C erhalten.
   IR (KBr): 3700-3320, 3935, 1705, 1655, 1600, 1515, 1265, 1115, 758 cm⁻¹.

### Beispiel 5

### 3-{5-[6-Carboxy-2-pyridyl-(1RS)-1-hydroxymethyl]-2-[6-(4-methoxyphenyl)-(5E)-5-hexenyloxy]-phenyl}-propionsäure

A. Eine Lösung von 100 mg 3-{5-[6-Methoxycarbonyl-2-pyridylcarbonyl]-2-[6-(4-methoxyphenyl)-(5E)-5-hexenyloxy]phenyl}-propionsäuremethylester in 5 ml Methanol wird unter Eiskühlung mit 10 mg Natriumborhydrid versetzt und 16 Stunden bei Raumtemperatur gerührt. Die Reaktionsmischung wird mit Wasser verdünnt, mit 1 n Schwefelsaure auf pH 6 angesäuert, mit Ethylacetat ausgeschüttelt, die organische Phase über Natriumsulfat getrocknet und eingeengt. Der Rückstand wird an Kieselgel mit Dichlormethan/Metahnol (0-2 %) chromatographiert. Es werden 58 mg 3-[5-⁸6-Methoxycarbonyl-2-pyridyl-(1RS)-1-hydroxymethyl⁹-2-⁸6-(4-methoxyphenyl)-(5E)-5-hexenyloxy⁹-phenyl]-propionsäuremethylester als farbloses Ol erhalten.
   IR (CHCl₃): 2925, 2855, 1730, 1610, 1505, 1440, 1248, 1040 cm⁻¹.
B. Unter den Bedingungen des Beispiels 3 D werden 9 mg 3-[5-⁸6-Methoxycarbonyl-2-pyridyl-(1RS)-1-hydroxymethyl⁹-2-⁸6-(4-methoxyphenyl)-(5E)-5-hexenyloxy⁹-phenyl]-propionsäuremethylester in 1 ml Methanol mit 0,5 ml 2 n Natronlauge verseift und aufbereitet. Es werden 8 mg der Titelverbindung als farbloses Ol erhalten.
   IR (CHCl₃): 3700-3140, 2930, 1720, 1610, 1518, 1250, 1120, 620 cm⁻¹.

### Beispiel 6

### 5-[3-(1-Carboxymethoxy)-4-⁸6-(4-methoxyphenyl)-(5E)-5-hexenyloxy⁹-phenyl]-5-oxo-pentansäure

A. Unter den Bedingungen des Beispiels 3 A werden 28,8 g Aluminiumchlorid in 320 ml Dichlormethan mit 11,9 g Glutarsäuremonomethylesterchlorid und 10 g Veratrol umgesetzt, aufbereitet und chromatographiert. Das so erhaltene Rohprodukt wird weiter unter den Bedingungen des Beispiels 3 A mit 270 g Pyridin, Hydrochlorid umgesetzt und aufbereitet. Es werden 5,6 g 5-(3,4-Dihydroxyphenyl)-5-oxo-pentansäure als Rohprodukt vom Schmelzpunkt 210-215 °C erhalten.
B. Eine Lösung von 5,6 g 5-(3,4-Dihydroxyphenyl)-5-oxo-pentansäure in 300 ml Methanol wird zusammen mit 15 g Amberlyst 15 16 Stunden bei Raumtemperatur gerührt. Die Reaktionsmischung wird über Kieselgur filtriert, eingeengt und der Rückstand an Kieselgel mit Hexan/Ethylacetat (20-60 %) chromatographiert. Es werden 3,7 g 5-(3,4-Dihydroxyphenyl)-5-oxo-pentansäuremethylester vom Schmelzpunkt 104-106 °C erhalten.
   IR (CHCl₃): 3480-3010, 2945, 1728, 1670, 1600, 1438, 1285, 1170 cm⁻¹.
C. Unter den Bedingungen des Beispiels 2 C werden 1,8 g 5-(3,4-Dihydroxyphenyl)-5-oxo-pentansäuremethylester in 19 ml Dimethylformamid in Gegenwart von 1,4 g Lithiumcarbonat mit 2,05 g (1E)-6-Brom-1-(4-methoxyphenyl)-1-hexen umgesetzt, aufbereitet und chromatographiert. Es werden 470 mg 5-{3-Hydroxy-4-[6-(4-methoxyphenyl)-(5E)-5-hexenyloxy]-phenyl}-5-oxo-pentansäuremethylester vom Schmelzpunkt 78-80 °C erhalten.
   IR (CHCl₃): 3680, 3620, 3020, 2395, 1730, 1510, 1420, 1215, 1040, 925 cm⁻¹.
D. Unter den Bedingungen des Beispiels 1 A werden 420 mg 5-{3-Hydroxy-4-[6-(4-methoxyphenyl)-(5E)-5-hexenyloxy]-phenyl}-5-oxo-pentansäuremethylester mit 167 mg Bromessigsäureethylester in 4 ml Dimethylformamid in Gegenwart von 650 mg Cäsiumcarbonat umgesetzt und aufbereitet. Es werden 562 mg 5-{3-(1-Ethoxycarbonylmethoxy)-4-[6-(4-methoxyphenyl)-(5E)-5-hexenyloxy]-phenyl}-5-oxo-pentansäuremethylester als farbloses Öl erhalten.
   IR (CHCl₃): 2940, 1752, 1733, 1675, 1600, 1510, 1250, 1200, 1150 cm⁻¹.
E. Unter den Bedingungen des Beispiels 3 D werden 256 mg 5-{3-(1-Ethoxycarbonylmethoxy)-4-[6-(4-methoxyphenyl)-(5E)-5-hexenyloxy]-phenyl}-5-oxo-pentansäuremethylester in 7 ml Methanol und 1 ml Tetrahydrofuran mit 4,3 ml 2 n Natronlauge verseift und aufbereitet. Es werden 105 mg der Titelverbindung vom Schmelzpunkt 128-130 °C erhalten.
   IR (CHCl₃): 3700-3000, 2960, 1705, 1680, 1600, 1515, 1430, 1250, 1140 cm⁻¹.

### Beispiel 7

### 5-[1-(3-Carboxyphenyl)-(1RS)-1-hydroxymethyl]-2-[6-(4-methoxyphenyl)-(5E)-5-hexenyloxy]-phenoxyessigsäure

Unter den Bedingungen des Beispiels 5 A werden 100 mg 5-(3-Carboxybenzoyl)-2-[6-(4-methoxyphenyl)-(5E)-5-hexenyloxy]-phenoxyessigsäure mit 31 mg Natriumborhydrid in 5 ml Dioxan und 0,5 ml Wasser umgesetzt und aufbereitet ()pH 3-4). Es werden 90 mg der Titelverbindung als farbloses Öl erhalten.
IR (CHCl₃): 3560-2980, 2960, 2845, 1710, 1603, 1502, 1245, 1115, 868 cm⁻¹.

## Patentansprüche

1. Leukotrien-B₄-Antagonisten der Formel I, 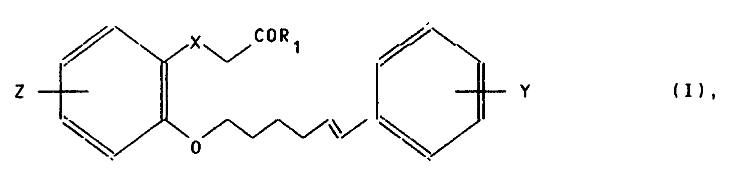 worin
X eine CH₂-Gruppe oder ein Sauerstoffatom,
Y C₁-C₄-Alkoxy oder -S(O)ₚ-(C₁-C₄)-Alkyl,
p 0, 1 oder 2
Z ein Wasserstoffatom oder den Rest-A-B-COOH mit A in der Bedeutung einer Hydroxymethylengruppe oder einer Carbonylgruppe und B in der Bedeutung einer Alkylengruppe mit 1 - 6 C-Atomen in der Kette oder eines Restes 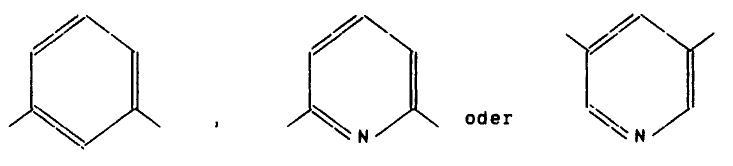 mit der Ausnahme, daß A nicht den Rest 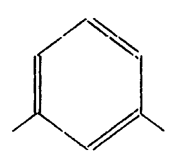
bedeutet, wenn X eine CH₂-Gruppe darstellt, R¹ den Rest OH, -O-(C₁-C₄)-Alkyl, -O-(C₃-C₆)-Cycloalkyl, -O-(C₆-C₁₀)Aryl, -O-(C₇-C₁₂)-Aralkyl oder den Rest NHR₄ mit R₄ in der Bedeutung Wasserstoff, (C₁-C₄)-Alkyl, (C₃-C₆)-Cycloalkyl oder (C₇-C₁₂)-Aralkyl darstellen sowie deren Salze mit physiologisch verträglichen Basen und deren Cyclodextrinclathrate.

2. Verfahren zur Herstellung von Leukotrien-B₄-Antagonisten der Formel I, dadurch gekennzeichnet, daß man in an sich bekannter Weise eine Verbindung der Formel II, 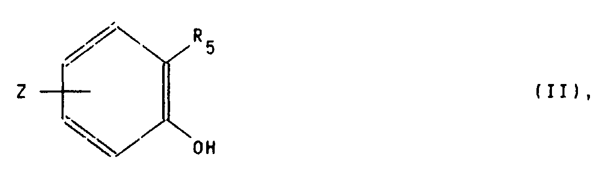 worin Z die oben angegenene Bedeutung hat und R₅ die Reste OH oder X-CH₂-COOR bedeutet, wobei X die oben angegebene Bedeutung hat und R eine C₁-C₄-Alkylgruppe darstellt, mit einer Verbindung der Formel III, 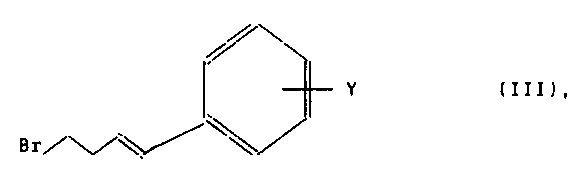 worin Y die oben angegebene Bedeutung hat, in Gegenwart von Cäsium-, Lithium- oder Kaliumcarbonat umsetzt und für den Fall, daß R₅ den Rest OH bedeutet mit einer Verbindung der Formel IV,
BrCH₂COOR (IV),
worin R die oben angegebene Bedeutung hat, umsetzt, gegebenenfalls die Carbonylgruppe A reduziert, Estergruppen verseift, Carboxylgruppen verestert oder erhaltenen Säuren der Formel I mit organischen oder anorganischen Basen oder Cyclodextrinen umsetzt.

3. Arzneimittel, bestehend aus einer oder mehreren Verbindungen gemäß Anspruch 1 und üblichen Hilfs- und Trägerstoffen.

## Claims

1. Leukotriene B₄ antagonists of formula I 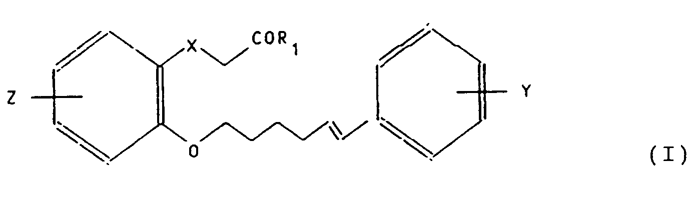 wherein
X represents a CH₂ group or an oxygen atom,
Y represents C₁-C₄-alkoxy or -S(O)ₚ-(C₁-C₄)-alkyl,
p is 0, 1 or 2,
Z represents a hydrogen atom or the radical A-B-COOH in which A represents a hydroxymethylene group or a carbonyl group and B represents an alkylene group having from 1 to 6 carbon atoms in the chain or a radical 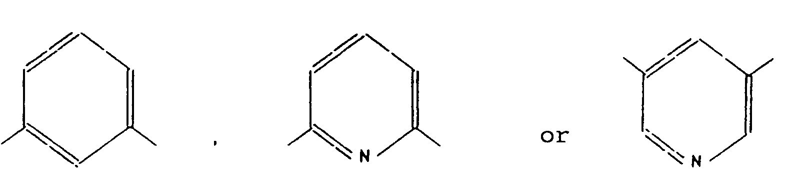 with the exception that A does not represent the radical 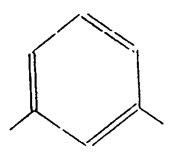 when X represents a CH₂ group,
R¹ represents the radical OH, -O-(C₁-C₄)-alkyl, -O-(C₃-C₆)-cycloalkyl, -O-(C₆-C₁₀)-aryl, -O-(C₇-C₁₂)-aralkyl or the radical NHR₄ in which R₄ represents hydrogen, (C₁-C₄)-alkyl, (C₃-C₆)-cycloalkyl or (C₇-C₁₂)-aralkyl,
and the salts thereof with physiologically tolerable bases and the cyclodextrin clathrates thereof.

2. Process for the preparation of leukotriene B₄ antagonists of formula I, characterised in that, in a manner known per se, a compound of formula II 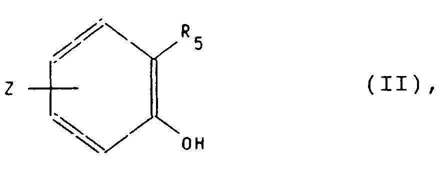 wherein Z has the meaning given above and R₅ represents the radical OH or X-CH₂-COOR in which X has the meaning given above and R represents a C₁-C₄-alkyl group, is reacted with a compound of formula III 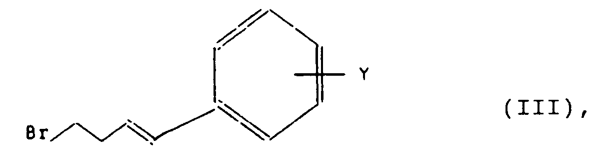 wherein Y has the meaning given above, in the presence of caesium, lithium or potassium carbonate, and in the case where R₅ represents the radical OH, is reacted with a compound of formula IV
BrCH₂COOR (IV),
wherein R has the meaning given above, optionally the carbonyl group A is reduced, ester groups are hydrolysed, carboxyl groups are esterified or resulting acids of formula I are reacted with organic or inorganic bases or cyclodextrins.

3. Medicament, consisting of one or more compounds according to claim 1 and customary excipients and carriers.

## Revendications

1. Antagonistes du leucotriène-B₄ de formule I ci-dessous 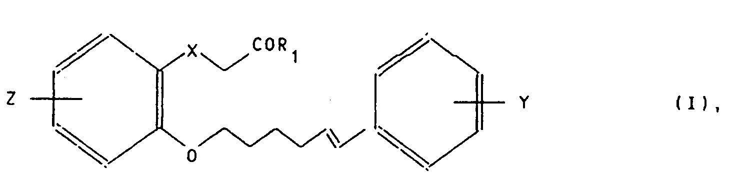 formule dans laquelle
X désigne un groupe CH₂ ou un atome d'oxygène,
Y un alcoxy en C₁-C₄ ou -S(O)ₚ-(alkyle en C₁-C₄),
p étant le nombre 0, 1 ou 2,
Z désigne un atome d'hydrogène ou un radical -A-B-COOH, A étant un groupe hydroxyméthylène ou un groupe carbonyle et B un groupe alkylène avec de 1 à 6 atomes de carbone dans la chaîne ou un radical 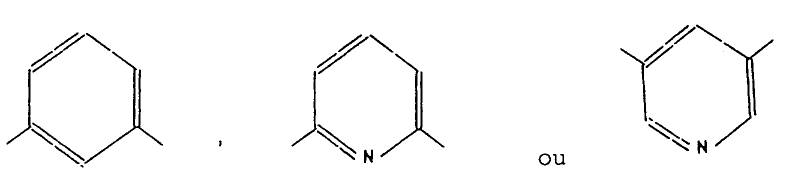 sauf que B ne peut être le radical 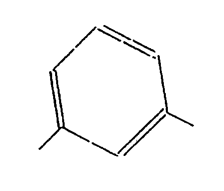 si X est un groupe CH₂, et
R¹ désigne un radical OH, -O-(alkyle en C₁-C₄), -O-(cycloalkyle en C₃-C₆), -O-(aryle en C₆-C₁₀), -O-(aralkyle en C₇-C₁₂), ou NHR₄, R₄ étant l'hydrogène ou un alkyle en C₁-C₄, cycloalkyle en C₃-C₆ ou aralkyle en C₇-C₁₂, ainsi que leurs sels formés avec des bases physiologiquement compatibles et leur clathrates de cyclodextrines.

2. Procédé de préparation des antagoniste du leucotriène-B₄ de formule I, caractérisé en ce que l'on fait réagir de manière en elle-même connue un composé de formule II 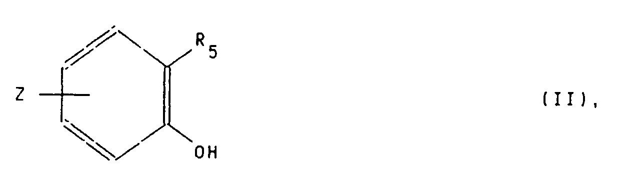 Z ayant la signification ci-dessus et R₅ désignant un radical OH ou X-CH₂-COOR, X ayant la signification ci-dessus et R étant un alkyle en C₁-C₄, avec un composé de formule III 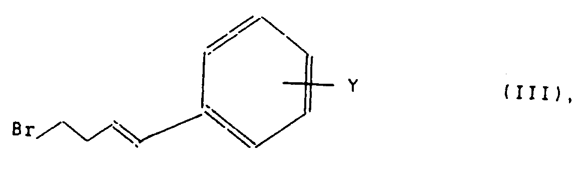 Y ayant la signification ci-dessus, en présence de carbonate de césium, de lithium ou de potassium, et si R₅ est le radical OH, on fait réagir avec un composé de formule IV
BrCH₂COOR (IV),
R ayant la signification ci-dessus, et le cas échéant on réduit le groupe carbonyle A, on saponifie des groupes d'esters, on estérifie des groupes carboxyliques ou on fait réagir les acides obtenus de formule I avec des bases organiques ou minérales ou des cyclodextrines.

3. Médicaments qui comprennent un ou plusieurs composés de la revendication 1 avec des adjuvants et véhicules courants.
